# EUROPEAN PATENT SPECIFICATION

(11) **EP 3 861 138 B1**
(45) Date of publication and mention of the grant of the patent: **04.03.2026**
(21) Application number: 19779497.7
(22) Date of filing: 03.10.2019
(51) Int. Cl.: C12Q 1/6886

(54) **METHOD FOR THE PROGNOSIS OF MELANOMA**
VERFAHREN ZUR PROGNOSE VON MELANOMEN
PROCÉDÉ DE PRONOSTIC DU MÉLANOME

(30) Priority: 03.10.2018 IT 201800009148
(43) Date of publication of application: 11.08.2021
(73) Proprietor: Alma Mater Studiorum - Università di Bologna, 40126 Bologna (BO) (IT)
(72) Inventor: DIKA, Emi, 40126 Bologna (BO) (IT); FERRACIN, Manuela, 40126 Bologna (BO) (IT); RIEFOLO, Mattia, 40126 Bologna (BO) (IT)
(74) Representative: Currado, Luisa
(86) International application number: PCT/EP2019/076823
(87) International publication number: WO 2020/070248

(56) References cited:
- WO-A1-2016/029260
- CN-A- 105 586 401
- LI JIANG ET AL: "The status of microRNA-21 expression and its clinical significance in human cutaneous malignant melanoma", ACTA HISTOCHEMICA, vol. 114, no. 6, 1 October 2012 (2012-10-01), pages 582 - 588, XP055184170, ISSN: 0065-1281, DOI: 10.1016/j.acthis.2011.11.001
- V GRIGNOL ET AL: "miR-21 and miR-155 are associated with mitotic activity and lesion depth of borderline melanocytic lesions", BRITISH JOURNAL OF CANCER, vol. 105, no. 7, 1 September 2011 (2011-09-01), GB, pages 1023 - 1029, XP055594478, ISSN: 0007-0920, DOI: 10.1038/bjc.2011.288
- M. RAIMO ET AL: "miR-146a Exerts Differential Effects on Melanoma Growth and Metastatization", MOLECULAR CANCER RESEARCH, vol. 14, no. 6, 1 June 2016 (2016-06-01), US, pages 548 - 562, XP055594472, ISSN: 1541-7786, DOI: 10.1158/1541-7786.MCR-15-0425-T
- D. PHILIPPIDOU ET AL: "Signatures of MicroRNAs and Selected MicroRNA Target Genes in Human Melanoma", CANCER RESEARCH, vol. 70, no. 10, 4 May 2010 (2010-05-04), pages 4163 - 4173, XP055009591, ISSN: 0008-5472, DOI: 10.1158/0008-5472.CAN-09-4512
- MUELLER D W ET AL: "miRNA expression profiling in melanocytes and melanoma cell lines reveals miRNAs associated with formation and progression of malignant melanoma", JOURNAL OF INVESTIGATIVE DERMATOLOGY, ELSEVIER, NL, vol. 129, no. 7, 1 July 2009 (2009-07-01), pages 1740 - 1751, XP002598392, ISSN: 0022-202X, [retrieved on 20090212], DOI: 10.1038/JID.2008.452
- N. LIN ET AL: "Expression of microRNA-106b and its clinical significance in cutaneous melanoma", GENETICS AND MOLECULAR RESEARCH, vol. 14, no. 4, 9 December 2015 (2015-12-09), pages 16379 - 16385, XP055594309, DOI: 10.4238/2015.December.9.6

## Description

### FIELD OF THE INVENTION

The present invention relates to an in vitro or ex vivo method for determining the prognosis and/or staging of a melanoma in a subject comprising the steps of:
a) measuring the amount of at least one microRNA selected from the group consisting of: miR-146a-5p, miR-155-5p, miR-21-5p, miR-363-5p and miR-584-5p in an isolated biological sample obtained from the subject and
b) determining the value of the Breslow thickness by regression analysis.

The present invention also relates to a kit for carrying out said method.

### PRIOR ART

The prevalence of cutaneous melanoma (CM) has risen in the past decade, reaching an annual increase of 3-7%. CM represents 3-5% of skin cancers. The majority of melanomas are diagnosed at an early stage and are treatable with surgical therapy (Glazer et al., 2016).

The main prognostic factor in CM is the Breslow thickness (Breslow, 1970). This parameter was introduced in 1970 by Alexander Breslow, who demonstrated that thickness influences the prognosis of CM. According to his study, in fact, melanomas with a thickness of less than 0.76 mm rarely metastasise.

The Breslow thickness (BT) is measured from the top of the granular layer of the epidermis (or from the base of the ulcer) to the base of the tumour. The thickness is measured by means of an ocular micrometer and the value must be reported, by approximation, to the first decimal place (Scolyer et al., 2013). It has in fact been demonstrated that the second decimal place is not reliable, hence it is necessary to approximate to the first decimal place.

Over the past 50 years, numerous studies have proposed clinical, histological or diagnostic imaging alternatives with the aim of replacing the Breslow thickness, but none of these parameters has ever succeeded in supplanting BT.

To date, the American Joint Committee on Cancer (AJCC, 8th edition) has confirmed the use of BT for staging and prognostic categorisation of melanoma (Gershenwald and Scolyer, 2018). The number of mitoses and ulceration represent another two parameters that have been studied as possible prognostic factors in association with BT; however, only ulceration has been demonstrated to have a role in prognosis, so much so that it is still used in association with the Breslow thickness in the 8th edition of the AJCC.

The Breslow thickness influences the subsequent staging of the patient with a sentinel lymph node biopsy. This diagnostic investigation is recommended, in fact, only in patients with a primary tumour thickness of >0.8 mm (Wong et al., 2018).

In the past 50 years, the Breslow thickness has been the most widely used parameter to determine the staging and follow-up of patients; however, this parameter shows limitations due to the variability among different operators in the interpretation of the measurement. These limitations and the consequent variability that has been reported can influence the choices of the clinician and the surgeon in the patient's management (Niebling et al., 2013; Elder, 2015; Xiao et al., 2016; Elmore et al., 2017; Taylor et al., 2018).

In the past 15 years, the deregulation of small RNA regulators, called microRNAs (miRNAs), in human tumours, including melanoma, has been documented (Mueller et al., 2009;Howell et al., 2010;Philippidou et al., 2010; Stark et al., 2010; Kozubek et al., 2013; Hanniford et al., 2015). According to the target they regulate and the tissue they are expressed in, microRNAs can have oncogenic or tumour suppressor roles. Oncogenic microRNAs, known as oncomiRs, exercise their role by targeting and blocking the activity of tumour suppressor genes. Other microRNAs, by contrast, may have a protective role, by negatively regulating the genes associated with tumour growth and metastasis. An imbalance between these two types of microRNA, in particular the overexpression of oncomiRs and underexpression of tumour suppressor microRNAs, influences the development and progression of the tumour (Negrini et al., 2007; Zhang et al., 2007).

The proliferation of melanoma cells can be influenced by microRNAs; in fact, the deregulation of some miRNAs can sustain and induce proliferative signals or else repress growth suppression pathways, thus promoting the carcinogenesis of melanoma. Furthermore, miRNAs can influence proliferation by regulating the proteins involved in controlling the cell cycle.

An alteration in the expression of specific miRNAs in melanoma can play a role as a prognostic marker. As described in Jiang et al., 2012, there are significant differences in the expression of miR-21 between dysplastic nevi and metastatic cutaneous melanomas and between primary melanomas and metastatic cutaneous melanomas. The overexpression of miR-21 can play a critical role in the progression of melanoma. In Jiang et al., 2012, however, no regression analysis was performed and no linear correlation was shown between the amount of microRNAs and the Breslow thickness.

Galasso et al. demonstrated that the loss of miR-204 is greater in melanomas with an NRAS mutation, whereas it is less frequent in those with a CDKN2A mutation. Furthermore, miR-204 was associated with a better prognosis in two independent cohorts of patients with melanoma; the exogenous expression thereof leads to an impairment of the growth of melanoma cell lines (Galasso et al., 2018).

Li Jang et al, Acta Histochemica 2012 discloses the correlation between miR-21 expression with clinicopathological factors or prognosis of patients with cutaneous melanoma. In particular, high miR-21 expression was found to be correlated with Breslow thickness and advanced clinical stage based on a statistical analysis that test the association between variables (Mann-Whitney's U test).

V. Grignol et al, British Journal of Cancer, 2011 explores the expression of miR-21 and miR-155 in primary melanoma lesions and borderline lesions in comparison with benign naevi.

M. Raimo et al, Molecular Cancer Research 2016 discloses that miR-146a exerts a dual role in melanoma cells, favoring tumor cell growth, while inhibiting cell invasion and presents the molecular mechanisms coordinated by miR-146a during melanoma malignancy that need to be considered for any therapeutic intervention.

WO2016/029260 A1 discloses determining the expression levels of miRNA-4487, miRNA-4706 and miRNA-4731 to evaluate the prognosis of melanoma.

Therefore, at present there is no available method which, by measuring the amount of microRNAs, makes it possible to obtain the value of the Breslow thickness of a melanoma indirectly, and thus to predict the prognosis of the patient.

Thus, there still exists a need for a method based on biomolecular markers that is capable of providing information on the Breslow thickness of melanomas, and hence on the prognosis of the patient.

### SUMMARY OF THE INVENTION

The present authors verified the expression of five specific microRNAs (miR-146a-5p, miR-155-5p, miR-21-5p, miR-363-5p and miR-584-5p) in relation to the Breslow thickness (BT) in primary cutaneous melanomas, with the aim of proposing a molecular surrogate of BT, to be used above all in thin melanomas.

### DETAILED DESCRIPTION OF THE INVENTION

The subject matter of the invention thus relates to an in vitro or ex vivo method for determining the prognosis and/or staging of a melanoma in a subject, comprising the steps of:
a) measuring the amount of:
   i. at least miR-21-5p and miR-146a-5p and optionally miR-155-5p or
   ii. at least miR-146a-5p and miR-155-5p and optionally miR-21-5p or
   iii. at least miR-21-5p and miR-155-5p and optionally miR-146a-5p in an isolated biological sample obtained from the subject and
b) determining the value of the Breslow thickness by regression analysis.

wherein the determination of the value of the Breslow thickness in step b) is performed by linear regression analysis by plotting the amount of at least one microRNA measured in step a) on a linear regression line of a graph of the amount of microRNA with respect to known values of the Breslow thickness and
wherein the value of the Breslow thickness determined in step b) is indicative of the prognosis of the patient and/or correlates with the degree of aggressiveness of the melanoma and/or wherein the patient is a patient who has been diagnosed with a melanoma or a patient who has not been given a certain staging.

Said at least one microRNA is preferably selected from the group consisting of: miR-146a-5p, miR-155-5p and miR-21-5p.

A further object of the invention is an in vitro or ex vivo method for determining the prognosis and/or staging of a melanoma in a subject, comprising the steps of:
a) measuring the amount of
   i. at least miR-21-5p and miR-146a-5p and optionally miR-155-5p or
   ii. at least miR-146a-5p and miR-155-5p and optionally miR-21-5p or
   iii. at least miR-21-5p and miR-155-5p and optionally miR-146a-5p in an isolated biological sample obtained from the subject and
b) determining the value of the Breslow thickness by regression analysis,

wherein the determination of the value of the Breslow thickness in step b) is performed by linear regression analysis by plotting the amount of at least one microRNA measured in step a) on a linear regression line of a graph of the amount of microRNA with respect to known values of the Breslow thickness and
wherein the value of the Breslow thickness determined in step b) is indicative of the prognosis of the patient and/or correlates with the degree of aggressiveness of the melanoma and/or wherein the patient is a patient who has been diagnosed with a melanoma or a patient who has not been given a certain staging.

Step a) may further comprises measuring the amount of miR-363-5p and/or miR-584-5p.

In the above defined methods, step a) preferably comprises measuring the amount of:
i. at least one microRNA selected from the group consisting of: miR-146a-5p and miR-155-5p and
ii. miR-21-5p.

More preferably, step a) comprises measuring the amount of miR-146a-5p and miR-21-5p, optionally further comprising measuring the amount of miR-155-5p and/or miR-363-5p and/or miR-584-5p.

Step a) preferably comprises measuring the amount of the three microRNAs: miR-146a-5p, miR-155-5p and miR-21-5p.

In another embodiment, step a) preferably comprises measuring the amount of the five microRNAs: miR-146a-5p, miR-155-5p, miR-21-5p, miR-363-5p and miR-584-5p.

Any combination of the above microRNA is included in the present invention. E.g. step a) may comprise measuring the amount of one, two, three, four (in any combination) or five of the above disclosed microRNAs.

The determination of the thickness value is preferably performed by plotting the amount of at least one microRNA measured in step a) on a linear regression line of a graph of the amount of microRNA with respect to known values of the Breslow thickness.

The value of the Breslow thickness determined in step b) is preferably indicative of the prognosis of the patient and/or correlates with the degree of aggressiveness of the melanoma. The correlation of the values of the Breslow thickness with the prognosis of the patient is described in the guidelines on melanoma (see, for example, Amin MB, Greene FL, Edge SB, et al. The Eighth Edition AJCC Cancer Staging Manual: Continuing to build a bridge from a population-based to a more "personalized" approach to cancer staging. CA Cancer J Clin 2017;67:93-9; Dummer R, Hauschild A, Lindeblatt N, et al. Cutaneous melanoma: ESMO Clinical Practice Guidelines for diagnosis, treatment and follow-up. Annals of Oncology 2015;26(5):v126-v132 (ESMO Guidelines also available online)).

In a preferred aspect of the invention, the patient is a patient who has been diagnosed with a melanoma or a patient who has not been given a certain staging.

The biological sample is preferably a tissue sample; preferably said sample is a formalin-fixed tissue embedded in paraffin or a fresh tissue.

Said melanoma is preferably a primary cutaneous melanoma, a melanoma associated with regression, a superficial spreading melanoma (SSM), or a melanoma with an associated nevus, and/or said method is preferably used to determine the stage of the melanoma.

In a preferred aspect of the method, the measurement of the amount of microRNA(s) is determined with a method comprising: RNA reverse transcription and / or nucleic acid hybridization and / or nucleic acid amplification and / or a combination thereof. The hybridization of the nucleic acids is preferably carried out using primers and/or probes, each of which is specific and selective for the sequence of one of the microRNAs defined above.

The amplification (and possible hybridization) of the nucleic acids is preferably carried out by quantitative real-time or digital PCR, more preferably comprising forward and reverse primers and optionally a probe.

In the method according to the invention the probe preferably comprises a sequence complementary to the sequences of the at least one miRNA as defined above.

The value of the Breslow thickness can also be understood here as "Breslow thickness equivalent" because the determination and quantification of the miRNA(s) could have value in themselves and be used in the place of BT.

In the present invention, the regression analysis that was performed is a linear regression analysis. However, the exact determination of the reference equations for calculating the Breslow thickness equivalent could be improved with an increase in the number of case studies. The person skilled in the art will be capable of performing such analyses and using the most appropriate statistical method. In a preferred aspect of the invention, the linear regression and the Pearson correlation coefficient r value were calculated by analysing, as variables, the normalised expression of the miRNA(s) and the Breslow thickness. The equation of the linear regression was subsequently used to estimate the thickness of the sample.

In a preferred embodiment, the equations of the linear regression lines are determined by regression analysis. Preferably, said equations are: y = 3.034x-0.6162 for miR-21-5p, y = 0.5733x-0.1037 for miR-146a-5p, y = 0.1442x-0.002872 for miR-155-5p, y = 1.240x - 0.2011 (R2 = 0.7715, R = 0.8783, p < 0.0001) for the mean expression of said three microRNAs.

The equations are preferably then used to obtain a prediction of the Breslow thickness in the patient.

In a preferred embodiment of the method of the invention, step a) comprises detecting and measuring the amount of all three or five microRNAs.

The method according to the invention preferably further comprises histopathological observation of the biological sample. Preferably, therefore, the method comprises a combination of clinical observation, histopathological observation and measurement of the levels of expression of the miRNAs in order to obtain a score that reflects the type of melanoma and the prognosis thereof.

The amounts of miRNAs measured preferably correspond to levels of expression that are normalised, preferably with respect to the level of expression of RNU44. The evaluation of an increased probability that the melanoma will progress preferably involves detecting the levels of said microRNA in said sample and calculating a prognostic score based, for each microRNA, on a predetermined equation using a predefined dataset. The measurement of the amount of miRNA is preferably performed by nucleic acid amplification and hybridization with primers and/or probes, each of which is specific and selective for the sequence of one of the microRNAs, preferably by qRT-PCR. Any other method for the detection and quantification of nucleic acids, such as digital PCR, microarrays or sequencing, is comprised within the scope of the invention.

The method according to the invention preferably comprises a step of extracting RNA from the biological sample. The RNA used to measure the levels of expression of the above-mentioned microRNAs is preferably extracted from a tissue sample, for example a biopsy or surgical piece, either fixed and embedded in paraffin or frozen or fresh, or a fluid.

In the kit detection means are understood to be sequence-specific amplification means and/or means for the quantitative detection of said amplified nucleic acids. In the context of the present invention the detection means are preferably specific primers and/or probes for each miRNA to be detected. Optionally, the kit according to the invention comprises control means. A further aspect of the invention relates to a microarray or a PCR reaction plate for carrying out the method as described above, comprising specific probes for each miRNA to be detected.

It is disclosed a kit for carrying out the above-mentioned methods, comprising
- means for detecting and / or measuring the amount of at least one microRNA as defined above and optionally,
- control means.

It is disclosed a kit for detecting and / or measuring the amount of at least one microRNA as defined above, consisting of:
- for each of said microRNAs, sequence-specific amplification means;
- means for the quantitative detection of said amplified nucleic acids;
- appropriate reagents.

It i s disclosed a device for measuring the amount of at least one miRNA as defined above in a biological sample, wherein said device consists of:
- solid support means, e.g. a microfluidic device, and
- a system for detecting the amount of microRNA.

Said device is preferably a chip microarray, a microfluidic printed circuit board, QPCR tubes, QPCR tubes in a strip or a QPCR plate.

In the method of the invention, when the level of expression of the miRNA corresponds to a value that is useful for staging according to the guidelines on melanoma, the prognosis of the patient will be more or less poor and/or the melanoma will have high or low probabilities of metastasising.

In the context of the present invention, the term "detection" can also be understood as "measurement of the amount". In the present invention, the expression "measurement of the amount" can be understood as a measurement of the amount or concentration or level of the respective miRNA and/or the DNA thereof, preferably semi-quantitative or quantitative. The term "amount", as used in the description, refers to, but is not limited to, the absolute or relative amount (or the level of concentration or expression) of miRNA and/or the DNA thereof, and any other value or parameter associated therewith or which can result therefrom. Methods for measuring miRNA and DNA in samples are well known in the art. For the purpose of detecting and/or measuring the levels of nucleic acid, the cells of the isolated biological sample can be lysed and the levels of miRNA in the lysates or purified or semi-purified RNAs from the lysates can be measured with any method known to the expert. Such methods include hybridisation assays that use detectable marked DNA or RNA probes (for example Northern blotting) and/or nucleic acid amplification, for example quantitative or semi-quantitative RT-PCR methods, using appropriate oligonucleotide primers, e.g. LNA primers. The person skilled in the art knows how to design the appropriate primers. Alternatively, quantitative or semiquantitative in situ hybridization assays can be performed using, for example, tissue sections, or undried cell suspensions, and marked, detectable DNA or RNA probes (for example, fluorescent or marked with the enzyme). Further methods for the quantification of miRNA include digital PCR, small RNA sequencing and microRNA microarrays.

The methods of the invention can further comprise normalisation of the levels of expression of miRNA. Normalisation includes, but is not limited to, regulation of the levels of expression of miRNA with respect to the levels of expression of one or more nucleic acids in the isolated biological sample.

Although the tested miRNAs are indicated as RNA sequences, it will be understood that when reference is made to hybridisations or to other assays, corresponding DNA sequences can be used. For example, the RNA sequences can be reverse transcribed and amplified using a polymerase chain reaction (PCR) to facilitate detection. In these cases, DNA rather than RNA will actually be directly quantified. It will also be understood that the complementary strand of the reverse transcribed DNA sequences can be analysed rather than the sequence itself. In this context, the term "complementary" refers to an oligonucleotide that has an exactly complementary sequence, i.e. for every adenine there is a thymine, etc. Although the assays can be performed individually for the miRNAs, it is generally preferable to assay various miRNAs or compare the ratio of two or more miRNAs.

In the kit "control means" are preferably used to compare the amount of microRNA with an appropriate control or an appropriate control amount. The "means for detecting and/or measuring the amount of microRNA" are known to the person skilled in the art and are preferably at least one marked, identifiable DNA or RNA probe specific for the miRNAs defined above and/or miRNA-specific primers for reverse transcribing or amplifying each of the aforesaid detected miRNAs. For example, said means can be specific TaqMan probes.

In the kit the sequence-specific amplification means are known to the person skilled in the art and are preferably at least one DNA or RNA primer, e.g. a "stem-loop RT primer" or an LNA primer.

The design of probes or primers specific for miRNA is known to the person skilled in the art and appropriate probes and/or primers can be commercially available for purchase.

The kit can further comprise appropriate reagents, such as, for example, an enzyme for the preparation of cDNA (e.g. reverse transcriptase) and/or PCR amplification (e.g. Taq polymerase) and /or a reagent for detecting and/or quantifying miRNA. Moreover, the kit can further comprise a reagent for the isolation of miRNA from samples and/or one or more normalisation controls. The normalisation control can be provided, for example, as one or more separate reagents for marking the samples or the reactions. The normalisation control(s) is/are preferably selected from endogenous RNA or miRNA expressed in the sample.

The kit comprises instructions for the interpretation of the data obtained.

According to the invention, the amount of microRNA is preferably determined by detecting a nucleic acid comprising, respectively, SEQ ID NO: 1, SEQ ID NO: 2, SEQ ID NO: 3, SEQ ID NO: 4, SEQ ID NO: 5, a variant or isoform thereof or fragments thereof.

The nucleic acid variants can include nucleic acid sequences that have about 75%-99.9% of identity, in terms of nucleic acid sequence, with a nucleic acid sequence described here. Preferably, a variant nucleic acid sequence will have at least about 75%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.7%, 99.8% or 99.9% of nucleic acid sequence identity with respect to a nucleic acid sequence of an entire length or a fragment of a nucleic acid sequence as described here.

The term "fragments" comprises nucleic acid sequences that may be truncated at the 5' end or 3' end, or may lack internal residues but maintain their function. Fragments are preferably from 18 to 24 nt long.

In the present invention, reference is made indistinctly to microRNA, miRNA or hsa-miR.

The miRNAs mentioned in the present invention include the sequences shown below (SEQ ID No. 1-5) and the homologues and analogues, the miRNA precursor molecules, the DNA molecules that encode said miRNAs and complementary nucleic acids.

### MicroRNA sequences:

miR-21-5p (SEQ ID NO: 1) >hsa-miR-21-5p MIMAT0000076 UAGCUUAUCAGACUGAUGUUGA
miR-146a-5p (SEQ ID NO: 2) >hsa-miR-146a-5p MIMAT0000449 UGAGAACUGAAUUCCAUGGGUU
miR-155-5p (SEQ ID NO: 3) >hsa-miR-155-5p MIMAT0000646 UUAAUGCUAAUCGUGAUAGGGGUU
miR-363-5p (SEQ ID NO: 4) >hsa-miR-363-5p MIMAT0003385 CGGGUGGAUCACGAUGCAAUUU
miR-584-5p (SEQ ID NO: 5) >hsa-miR-584-5p MIMAT0003249 UUAUGGUUUGCCUGGGACUGAG

The identity of a homologue to a sequence of SEQ ID NO: 1-5 can preferably be at least 90%, more preferably at least 95% identical, up to 99.9%.

The present invention will now be illustrated with non-limiting examples in reference to the following figures.
**Figure 1****.** Correlation between BT and expression of miR-21-5p. (a) All samples included in the study. (b) the same curve enlarged for BT values of 0-1.5 mm.
**Figure 2****.** Correlation between BT and expression of miR-146a-5p. (a) All samples included in the study. (b) the same curve enlarged for BT values of 0-1.5 mm.
**Figure 3****.** Correlation between BT and expression of miR-155-5p. (a) All samples included in the study. (b) the same curve enlarged for BT values of 0-1.5 mm.
**Figure 4****.** Correlation between BT and the mean expression of miR-21-5p, miR-146a-5p and miR-155-5p. (a) the curve of all samples included in the study. (b) the same curve enlarged for BT values of 0-1.5 mm.
**Figure 5****:** Correlation between BT and expression of miR-21-5p, miR-146a-5p in Superficial Spreading Melanoma (a, b) and in Nodular Melanoma (c, d). Correlation between BT and the mean expression of miR-21-5p and miR-146a-5p in Superficial Spreading Melanoma (e)

### DETAILED DESCRIPTION OF THE INVENTION

### Materials and Methods

### Example 1

### Patients

Formalin-fixed paraffin-embedded (FFPE) samples from patients with a histological diagnosis of melanoma were collected and stored in the dermatopathology laboratory of the Bologna University Hospital. All subjects involved gave their informed consent to participation in the study. The study was conducted in accordance with the principles of the Declaration of Helsinki and the protocol was approved by the Ethics Committee of the "Area Vasta Emilia Romagna Centro - Italia (417/2018/Sper/AOUBo)". Primary cutaneous melanomas were included in the study.

Exclusion criteria against participation in the study were: the presence of regression at the histological examination, melanoma with an associated nevus, ulcerations, and the presence of numerous mitotic figures (>1). These cases were excluded in order to avoid any confusing factors. The selection of patients, samples and sections stained with hematoxylin and eosin was made by a dermatologist. The samples had a Breslow thickness comprised between 0.2 mm and 4 mm. All cases were evaluated blindly by two independent pathologists; cases without agreement between the two pathologists were excluded.

### RNA extraction

Five 10 µm sections of FFPE tissue were obtained for each patient. Furthermore, a 3 µm section was stained with hematoxylin and eosin in order to identify and select the area morphologically containing the tumour cells. The RNA of the tumour cells was isolated using the miRNeasy kit (Qiagen), according to the manufacturer's instructions. The yield of RNA was checked by measuring absorbance at 260-280 nm with a NanoDrop-1000 spectrophotometer (NanoDrop-Technologies, Wilmington, USA).

### Analysis of microRNA expression with quantitative PCR

After the extraction of total RNA, 10 ng of RNA were reverse transcribed in a 10 µL reaction using the Universal cDNA synthesis kit (Qiagen) and following the manufacturer's instructions. Quantitative PCR for miR-21-5p, miR-155-5p, miR-146a-5p and RNU44, used as the gene of reference, was performed in a reaction volume of 10 µL, with the addition of 1 µL of miRCURY LNA primer mix, 5 µL of miRCURY LNA SYBR Green PCR mix and 4 µL of cDNA of the sample. All samples were analysed in triplicate using the following thermal cycle conditions: 95 °C for 10 minutes and 39 cycles of 95 °C for 10 seconds, 60 °C for 1 minute. The data obtained from the quantitative PCR were analysed with the software application Bio-Rad CFX Manager. For each miRNA in each sample, a threshold cycle (Ct) was determined, which was inversely proportional to the amount of the initial model. The normalised expression of the miRNA was calculated using RNU44 as the gene of reference with the method 2^{-(ΔCt)} (Livak and Schmittgen, 2001).

### Statistical analysis

The linear regression line, r² and the Pearson correlation coefficient r value were calculated analysing as variables the normalised expression of the miRNA and the Breslow thickness, using Graphpad Prism 6 (GraphPad Software). The equation of the linear regression was subsequently used to estimate the thickness of the sample.

### Results

35 patients with a histological diagnosis of melanoma were enrolled. The mean Breslow thickness was 0.73 ± 0.82 mm (interval 0.2 - 4.0); the Breslow thickness of the individual patients is shown in **Table 1.**

Inventors tested the expression of miR-21-5p, miR-146a-5p and miR-155-5p by RT-qPCR with miRCURY LNA assays (Qiagen, ex Exiqon), normalising each miRNA using RNU44 as the reference gene. This value was then correlated with the parameter BT.

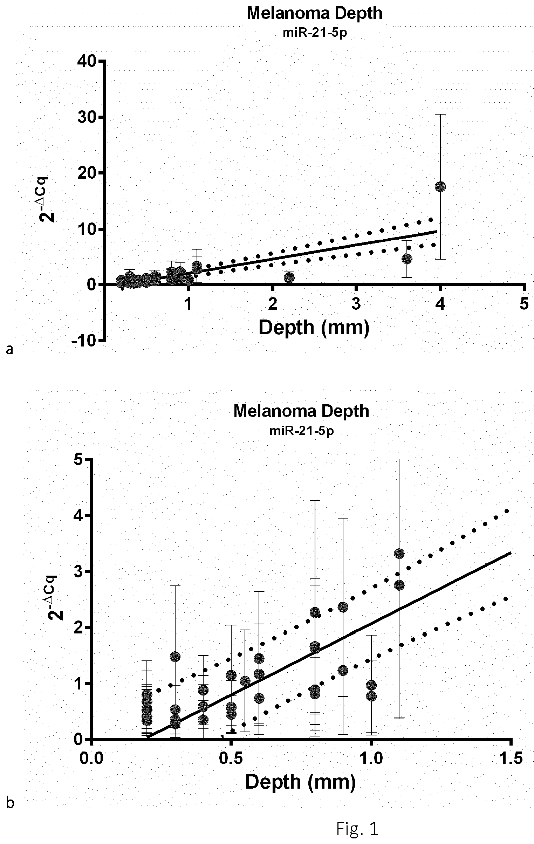

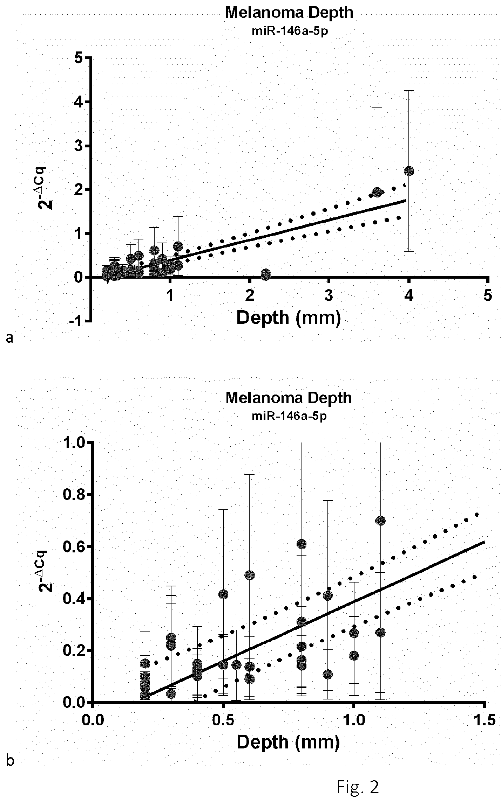

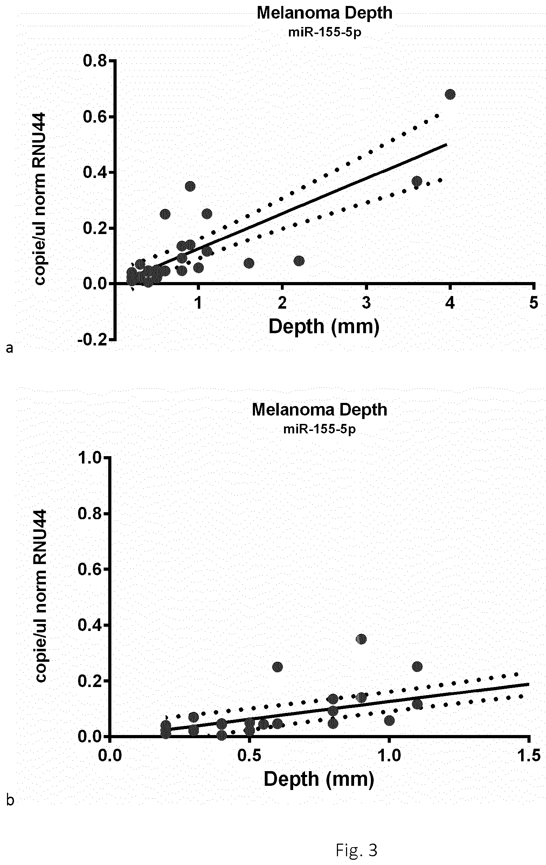

The expression of each of the three miRNAs in the present case study has a significant Pearson's correlation (p <0.0001) with BT. More specifically, miR-21-5p has R = 0.85 and R2 = 0.59944, miR-146a-5p has R = 0.95, R2 = 0.6741, miR-155-5p has R = 0.87, R2 = 0.6438, as shown in **Figure 1-3****.**

Regression analysis was then used to determine the equations of the linear regression lines, which were y = 3.034x-0.6162 for miR-21-5p, y = 0.5733x-0.1037 for miR-146a-5p and y = 0.1442x-0.002872 for miR-155-5p.

The three equations were subsequently used to obtain a prediction of the Breslow thickness in our group of patients from each individual miRNA. The mean of the three values was also calculated (**Table 1**)**.**

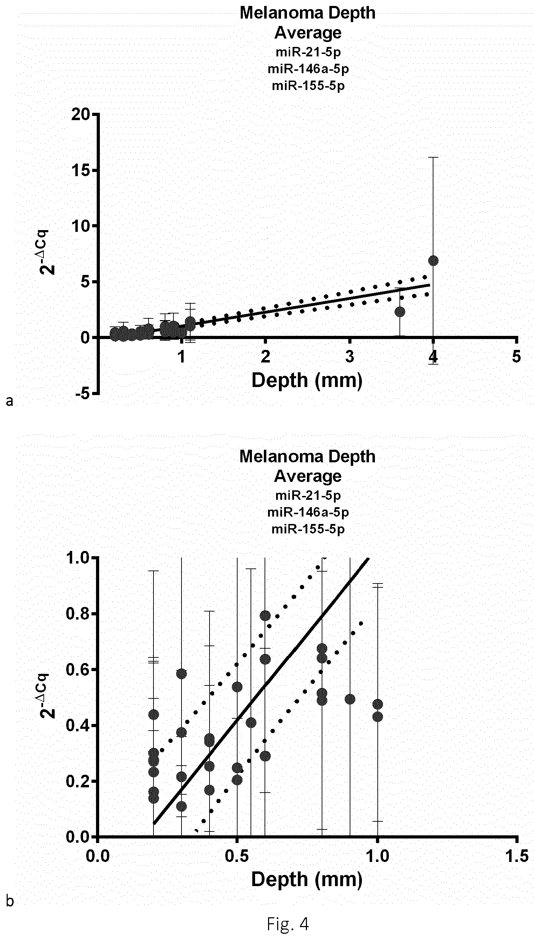

Regression analysis was also used to determine the equation of the linear regression line for the mean expression of the three microRNAs, i.e. y = 1.240x - 0.2011 (R2 = 0.7715, R = 0.8783, p < 0.0001) (see **Figure 4**).

The present analysis clearly demonstrates the possibility of integrating, or considering analogous, the physical determination of BT with a molecular analysis based on an evaluation of miRNA(s).

### Discussion

The first to demonstrate the importance of the histopathological characteristics of primary melanoma in determining the prognosis of the patient were Allan and Spitz in the 1950s. They proposed a simple classification with two categories: superficial and nodular melanomas. Clark et al. subsequently contributed to the first histopathological classification of melanoma (5 Clark levels). This classification was used for nearly two decades until Alexander Breslow presented his idea of measuring the thickness of melanoma as a quantitative parameter that would predict the clinical course. The first study that demonstrated the strength of his theory included only a small series of melanomas (n = 25). It took nearly a decade to validate the data of his micro-staging system and to have his assumptions accepted worldwide (World Health Organization - 1979). Today, this discovery has been considered one of the most important in research on melanoma. Various studies demonstrate that BT remains the most important parameter for melanoma staging (Gershenwald and Scolyer, 2018).

The aim of this study is to propose a molecular prognostic parameter that can be a supplement or alternative to the evaluation of the Breslow thickness.

At present, in fact, there are no alternative methods for determining BT or, consequently, of staging and determining the aggressiveness of the melanoma in the event of failure of the traditional method. This frequently leads to cases of over-diagnosis by physicians, implying the use of invasive diagnostic procedures during staging and management of the patient in the follow-up.

Furthermore, the BT measurement has many limitations:
1) It is a histopathological parameter measured by the "human eye" with a micrometer (a physical parameter) which thus generates an inevitable variability among observers.
2) There are conditions in which it is impossible to determine the exact BT, as in melanoma associated with regression (10-20% of the cases), and melanoma arising from a nevus (up to 40% of the cases). In all these cases (60% of all melanomas), determination of the Breslow thickness is very difficult, or it is impossible to define. In fact, the presence of mononucleated cells of macrophages, typical of regression, rule out the possibility for a pathologist to observe the extension of atypical melanocytes.

In the latter case, when the melanoma arises on pre-existing nevi, there exists a "grey zone" wherein the atypical melanocytes of melanoma are mixed with the melanocytes of the nevus (benign component) and tracing a line that separates the tumour from the nevus is not always possible.

The present method provides a standard curve designed to predict thickness in the melanoma.

Introducing the use of a molecular parameter (expression of miRNA) in cases in which a validation is necessary or when the pathologist is not able to determine BT with any confidence. More specifically, the present method can be used to overcome the difficulties explained in points 1 and 2, since what is involved is a molecular parameter that is not susceptible to variability among operators or to other confusing histopathological factors. In fact, it represents a non-subjective quantitative parameter.

The present method can be easily implemented by diagnostic laboratories and can help clinicians (pathologists, dermatologists, surgeons and oncologists) in the staging and clinical management of patients with melanoma who are not eligible for traditional evaluation.

**Table 1.**

| **Sample** | **Breslow thickness (pathologists)** | ***Mean estimate from the regression analysis*** | **Estimate of BT from the expression of miR-21-5p** | **Estimate of BT from the expression of miR-146a-5p** | **Estimate of BT from the expression of miR-155-5p** |
|---|---|---|---|---|---|
| MEL-02 | 0.8 | *1.0* | 0.9 | 1.2 | 1.0 |
| MEL-05 | 0.2 | *0.3* | 0.3 | 0.3 | 0.2 |
| MEL-06 | 0.6 | *0.4* | 0.4 | 0.3 | 0.3 |
| MEL-07 | 0.3 | *0.5* | 0.4 | 0.6 | nd |
| MEL-08 | 0.5 | *0.3* | 0.4 | 0.4 | 0.2 |
| MEL-09 | 0.5 | *0.3* | 0.4 | 0.4 | 0.2 |
| MEL-10 | 0.4 | *0.3* | 0.3 | 0.4 | 0.1 |
| MEL-11 | 0.2 | *0.3* | 0.4 | 0.4 | 0.2 |
| MEL-12 | 0.3 | *0.5* | 0.7 | 0.6 | 0.2 |
| MEL-13 | 0.2 | *0.2* | 0.3 | 0.3 | 0.1 |
| MEL-14 | 0.4 | *0.4* | 0.5 | 0.4 | 0.3 |
| MEL-15 | 1 | *0.5* | 0.5 | 0.6 | 0.4 |
| MEL-16 | 1.1 | *1.5* | 1.3 | 1.4 | 1.8 |
| MEL-17 | 3.6 | *2.6* | 1.7 | 3.6 | 2.6 |
| MEL-18 | 0.8 | *0.7* | 0.7 | 0.7 | 0.7 |
| MEL-19 | 0.5 | *0.6* | 0.6 | 0.9 | 0.4 |
| MEL-20 | 0.2 | *0.3* | 0.4 | 0.3 | nd |
| MEL-21 | 0.8 | *0.5* | 0.5 | 0.4 | nd |
| MEL-22 | 0.6 | *1.1* | 0.6 | 1.0 | 1.8 |
| MEL-23 | 0.8 | *0.5* | 0.8 | 0.6 | 0.3 |
| MEL-24 | 0.3 | *0.5* | 0.3 | 0.6 | 0.5 |
| MEL-28 | 0.9 | *0.7* | 0.6 | 0.4 | 1.0 |
| MEL-29 | 0.4 | *0.4* | 0.4 | 0.4 | nd |
| MEL-32 | 0.3 | *0.2* | 0.3 | 0.2 | 0.2 |
| MEL-33 | 0.4 | *0.4* | 0.4 | 0.4 | 0.3 |
| MEL-34 | 0.2 | *0.4* | 0.5 | 0.3 | nd |
| MEL-35 | 0.55 | *0.4* | 0.5 | 0.4 | 0.3 |
| MEL-36 | 0.2 | *0.3* | 0.4 | 0.2 | nd |
| MEL-37 | 0.8 | *0.5* | 0.5 | 0.5 | nd |
| MEL-38 | 1 | *0.6* | 0.5 | 0.5 | 0.8 |
| MEL-39 | 1.1 | *0.9* | 1.1 | 0.7 | nd |
| MEL-40 | 0.6 | *0.6* | 0.7 | 0.4 | nd |
| MEL-52 | 4 | *5.0* | 6.0 | 4.4 | 4.7 |
| MEL-56 | 0.9 | *1.4* | 1.0 | 0.9 | 2.4 |
| MEL-57 | 0.2 | *0.4* | 0.4 | 0.4 | 0.3 |
| *nd = not determined* | | | | | |

### Example 2

### Identification of microRNAs directly correlated with Breslow thickness from Next Generation Sequencing (NGS) data

Inventors analyzed the global miRNA profile of 21 primary melanoma samples and 3 benign nevi using a smallRNA sequencing approach and detected 1983 human miRNAs expressed in at least one sample.

Briefly, smallRNA libraries were prepared using TruSeq Small RNA Library PrepKit v2 (Illumina, RS-200-0012/24/36/48), according to manufacturer's indications. The library pool was sequenced using NextSeq 500/550 High Output Kit v2 (75 cycles) (Illumina, FC-404-2005) on Illumina NextSeq500 platform. Base-call data were demultiplexed by Illumina BaseSpace Sequence Hub and converted to FASTQ format. After a quality check, read mapping was performed using STAR algorithm. The reference genome was human microRNAs sequences from the miRbase v.21.

They compared thin (Breslow thickness < 0.7mm) and thick (Breslow thickness >= 0.8mm) tumors to identify miRNAs potentially correlating with BT and obtained a list of 50 miRNAs differentially expressed between the two groups (p<0.05, fold change >2), some of them were upregulated in the thick group, some were downregulated.

Among the miRNAs upregulated in the thicker tumors, inventors performed a correlation analysis to identify the microRNAs that were significantly correlated with BT. We used expression data and BT measure in mm to obtain Person r correlation index and the p-value of this correlation for each miRNA/BT pair (Table 2).

**Table 2. microRNAs significantly correlating with Breslow thickness in 21 primary melanomas**

| **miRNA** | **Pearson r** | **95% confidence interval** | **R square** | **P (two-tailed)** | **P value summary** |
|---|---|---|---|---|---|
| BT vs. hsa-miR-20b-5p | 0.9327 | 0.8301 to 0.9742 | 0.8699 | 0.0001 | **** |
| **BT vs. hsa-miR-363-3p** | **0.7834** | **0.5109 to 0.9128** | **0.6137** | 0.0001 | **** |
| **BT vs. hsa-miR-584-5p** | **0.8512** | **0.6472 to 0.9415** | **0.7246** | 0.0001 | **** |
| **BT vs. hsa-miR-146a-5p** | **0.7424** | **0.4347 to 0.8949** | **0.5511** | **0.0003** | *** |
| **BT vs. hsa-miR-21-5p** | **0.7369** | **0.4248 to 0.8924** | **0.543** | **0.0003** | *** |
| BT vs. hsa-miR-181a-3p | 0.7287 | 0.4101 to 0.8888 | 0.5309 | 0.0004 | *** |
| BT vs. hsa-miR-21-3p | 0.7254 | 0.4043 to 0.8873 | 0.5262 | 0.0004 | *** |
| **BT vs. hsa-miR-155-5p** | **0.6165** | **0.2252 to 0.8365** | **0.38** | **0.0049** | ** |

Importantly, only these microRNAs were linearly correlated with BT.

Inventors excluded miR-20b-5p and miR-21-3p for their low expression in tumor samples, and miR-181a-3p for its high expression in nevi.

Finally, inventors selected 5 miRNAs (in bold in Table 2) for a further validation in 120 melanoma samples.

### Example 3

### Validation data for miR-146a-5p and miR-21-5p in 120 samples

Inventors tested miR-146a-5p and miR-21-5p in a cohort of 120 samples from Bologna and Torino hospitals.

The strong positive correlation was confirmed for both miR-146a-5p and miR-21-5p in the most frequent subtype of cutaneous melanoma, which is superficial spreading melanoma (SSM). Inventors tested 95 samples, obtaining significant results in term of direct correlation of each BT/miRNA pair (Table 3).

Specifically, inventors normalized each miRNA on two different reference genes (SNORD48 (Gene ID: 26801) and SNORD44 (Gene ID: 26806)). Inventors tested the average of these two assessments against BT and also the final average of the expression of the two miRNAs vs. BT. The average of two miRNAs provided the highest correlation index. This direct correlation was not observed in nodular melanoma (NM) subtype.

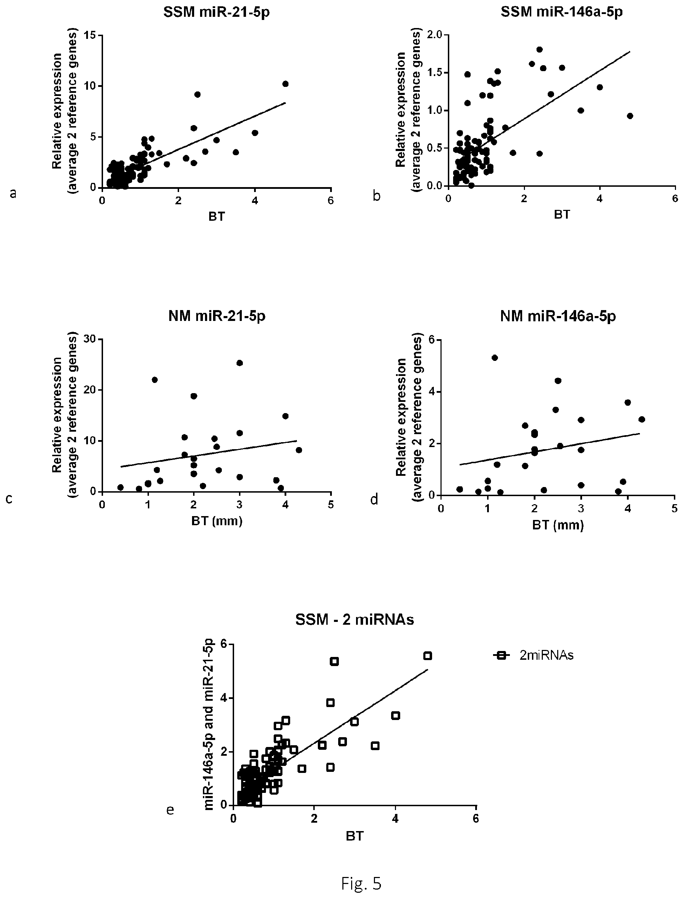

See Figure 5 for a graphical representation.

### Methods

(SSM): miR-21-5p and miR-146a-5p expression was assessed by RT-qPCR in 95 cases of superficial spreading melanoma (SSM, n=95). RNA was extracted from archival FFPE blocks collected at the Dermatopathology unit of the University of Bologna (60 cases) and Pathology Unit at the University of Turin (35 cases). Two reference small non-coding RNAs (SNORD44 and SNORD48) were used for expression normalization. All cases were blinded reviewed for Breslow thickness (BT) determination form two pathologists. BT range was 0.2-4.5 mm. All selected patients had at least 5 years of follow-up.

### Results

The normalized expression of miR-21-5p and miR-146a-5p significantly correlates with Breslow thickness in SSM: miR-21-5p/BT correlation r=0.79, p<0.0001; miR-146a-5p/BT r=0.62, p<0.0001. Inventors observed also that BT cut-off equivalent in miRNA expression, both for each miRNA and their combination, successfully identifies all relapsing SSM patients.

### Conclusions

miR-21-5p and miR-146a-5p expression significantly correlates with Breslow thickness in superficial spreading melanoma. The use of these miRNAs as molecular marker, in association with Breslow thickness measurement, can be proposed as an aid for pathologists when dealing with difficult cases.

**Table 3. Correlation analysis of miR-21-5p and miR-146a-5p, alone and in combination, with BT in 95 SSM.**

| | **BT vs. miR-21-5p/ SNORD44** | **BT vs. miR-21-5p/ SNORD48** | **BT vs. miR-21-5p average** | **BT vs. miR-146a-5p/ SNORD44** | **BT vs. miR-146a-5p/ SNORD48** | **BT vs. miR-146a-5p average** | **BT vs. 2miRNAs** |
|---|---|---|---|---|---|---|---|
| **Pearson r** | | | | | | | |
| **r** | 0.7298 | 0.7573 | 0.7914 | 0.4461 | 0.6269 | 0.6156 | **0.7979** |
| **95% confidence interval** | 0.6186 to 0.8123 | 0.6552 to 0.8323 | 0.7012 to 0.8567 | 0.2666 to 0.5957 | 0.4851 to 0.7366 | 0.4710 to 0.7281 | 0.7100 to 0.8613 |
| **R square** | 0.5326 | 0.5735 | 0.6264 | 0.199 | 0.393 | 0.379 | 0.6366 |
| **P value** | | | | | | | |
| **P (two-tailed)** | < 0.0001 | < 0.0001 | < 0.0001 | < 0.0001 | < 0.0001 | < 0.0001 | < 0.0001 |
| **P value summary** | **** | **** | **** | **** | **** | **** | **** |
| **Significant? (alpha = 0.05)** | Yes | Yes | Yes | Yes | Yes | Yes | Yes |

### REFERENCES

Breslow, A. (1970). Thickness, cross-sectional areas and depth of invasion in the prognosis of cutaneous melanoma. Ann Surg 172, 902-908.
Elder, D.E. (2015). Pathology of melanoma. Surg Oncol Clin N Am 24, 229-237.
Elmore, J.G., Barnhill, R.L., Elder, D.E., Longton, G.M., Pepe, M.S., Reisch, L.M., Carney, P.A., Titus, L.J., Nelson, H.D., Onega, T., Tosteson, A.N.A., Weinstock, M.A., Knezevich, S.R., and Piepkorn, M.W. (2017). Pathologists' diagnosis of invasive melanoma and melanocytic proliferations: observer accuracy and reproducibility study. BMJ 357, j2813.
Galasso, M., Morrison, C., Minotti, L., Corra, F., Zerbinati, C., Agnoletto, C., Baldassari, F., Fassan, M., Bartolazzi, A., Vecchione, A., Nuovo, G.J., of Leva, G., D'atri, S., Alvino, E., Previati, M., Nickoloff, B.J., Croce, C.M., and Volinia, S. (2018). Loss of miR-204 expression is a key event in melanoma. Mol Cancer 17, 71.
Gershenwald, J.E., and Scolyer, R.A. (2018). Melanoma Staging: American Joint Committee on Cancer (AJCC) 8th Edition and Beyond. Ann Surg Oncol 25, 2105-2110.
Glazer, A.M., Winkelmann, R.R., Farberg, A.S., and Rigel, D.S. (2016). Analysis of Trends in US Melanoma Incidence and Mortality. JAMA Dermatol.
Hanniford, D., Segura, M.F., Zhong, J., Philips, E., Jirau-Serrano, X., Darvishian, F., Berman, R.S., Shapiro, R.L., Pavlick, A.C., Brown, B., Osman, I., and Hernando, E. (2015). Identification of metastasis-suppressive microRNAs in primary melanoma. J Natl Cancer Inst 107.
Howell, P.M., Jr., Li, X., Riker, A.I., and Xi, Y. (2010). MicroRNA in Melanoma. Ochsner J 10, 83-92.
Jiang, L., Lv, X., Li, J., Li, J., Li, X., Li, W., and Li, Y. (2012). The status of microRNA-21 expression and its clinical significance in human cutaneous malignant melanoma. Acta Histochem 114, 582-588.
Kozubek, J., Ma, Z., Fleming, E., Duggan, T., Wu, R., Shin, D.G., and Dadras, S.S. (2013). In-depth characterization of microRNA transcriptome in melanoma. PLoS One 8, e72699.
Livak, K.J., and Schmittgen, T.D. (2001). Analysis of relative gene expression data using real-time quantitative PCR and the 2(-Delta Delta C(T)) Method. Methods 25, 402-408.
Mueller, D.W., Rehli, M., and Bosserhoff, A.K. (2009). miRNA expression profiling in melanocytes and melanoma cell lines reveals miRNAs associated with formation and progression of malignant melanoma. J Invest Dermatol 129, 1740-1751.
Negrini, M., Ferracin, M., Sabbioni, S., and Croce, C.M. (2007). MicroRNAs in human cancer: from research to therapy. J Cell Sci 120, 1833-1840.
Niebling, M.G., Haydu, L.E., Karim, R.Z., Thompson, J.F., and Scolyer, R.A. (2013). Reproducibility of AJCC staging parameters in primary cutaneous melanoma: an analysis of 4,924 cases. Ann Surg Oncol 20, 3969-3975.
Philippidou, D., Schmitt, M., Moser, D., Margue, C., Nazarov, P.V., Muller, A., Vallar, L., Nashan, D., Behrmann, I., and Kreis, S. (2010). Signatures of microRNAs and selected microRNA target genes in human melanoma. Cancer Res 70, 4163-4173.
Scolyer, R.A., Judge, M.J., Evans, A., Frishberg, D.P., Prieto, V.G., Thompson, J.F., Trotter, M.J., Walsh, M.Y., Walsh, N.M., Ellis, D.W., and International Collaboration on Cancer, R. (2013). Data set for pathology reporting of cutaneous invasive melanoma: recommendations from the international collaboration on cancer reporting (ICCR). Am J Surg Pathol 37, 1797-1814.
Stark, M.S., Tyagi, S., Nancarrow, D.J., Boyle, G.M., Cook, A.L., Whiteman, D.C., Parsons, P.G., Schmidt, C., Sturm, R.A., and Hayward, N.K. (2010). Characterization of the Melanoma miRNAome by Deep Sequencing. PLoS One 5, e9685.
Taylor, L., Hood, K., Reisch, L., Elmore, J., Piepkorn, M., Barnhill, R., Knezevich, S., Radick, A., and Elder, D. (2018). Influence of variability in assessment of Breslow thickness, mitotic rate and ulceration among US pathologists interpreting invasive melanoma, for the purpose of AJCC staging. J Cutan Pathol 45, 588-596.
Wong, S.L., Faries, M.B., Kennedy, E.B., Agarwala, S.S., Akhurst, T.J., Ariyan, C., Balch, C.M., Berman, B.S., Cochran, A., Delman, K.A., Gorman, M., Kirkwood, J.M., Moncrieff, M.D., Zager, J.S., and Lyman, G.H. (2018). Sentinel Lymph Node Biopsy and Management of Regional Lymph Nodes in Melanoma: American Society of Clinical Oncology and Society of Surgical Oncology Clinical Practice Guideline Update. Ann Surg Oncol 25, 356-377.
Xiao, D., Barry, S., Kmetz, D., Egger, M., Pan, J., Rai, S.N., Qu, J., Mcmasters, K.M., and Hao, H. (2016). Melanoma cell-derived exosomes promote epithelial-mesenchymal transition in primary melanocytes through paracrine/autocrine signaling in the tumor microenvironment. Cancer Lett 376, 318-327.
Zhang, B., Pan, X., Cobb, G.P., and Anderson, T.A. (2007). microRNAs as oncogenes and tumor suppressors. Dev Biol 302, 1-12.

## Claims

1. An in vitro or ex vivo method for determining the prognosis and/or staging of a melanoma in a subject comprising the steps of:
a) measuring the amount of:
i. at least miR-21-5p and miR-146a-5p and optionally miR-155-5p or
ii. at least miR-146a-5p and miR-155-5p and optionally miR-21-5p or
iii. at least miR-21-5p and miR-155-5p and optionally miR-146a-5p in an isolated biological sample obtained from the subject and
b) determining the value of Breslow thickness by regression analysis,
wherein the determination of the value of the Breslow thickness in step b) is performed by linear regression analysis by plotting the amount of at least one microRNA measured in step a) on a linear regression line of a graph of the amount of microRNA with respect to known values of the Breslow thickness and wherein the value of the Breslow thickness determined in step b) is indicative of the prognosis of
the patient and/or correlates with the degree of aggressiveness of the melanoma and/or wherein the patient is a patient who has been diagnosed with a melanoma or a patient who has not been given a certain staging.

2. The method according to claim 1, wherein step a) comprises measuring the amount of at least miR-21-5p and miR-146a-5p and optionally miR-155-5p.

3. The method according to the preceding claims, wherein step a) comprises measuring the amount of the three microRNAs: miR-21-5p, miR-146a-5p and miR-155-5p.

4. The method according to the preceding claims, wherein step a) comprises measuring the amount of the five microRNAs: miR-21-5p, miR-146a-5p, miR-155-5p, miR-363-5p and miR-584-5p.

5. The method according to any one of the preceding claims, wherein the biological sample is a tissue sample, preferably said sample is a formalin-fixed tissue and embedded in paraffin or a fresh tissue and/or wherein the melanoma is a primary cutaneous melanoma, a regression associated melanoma, a superficial spreading melanoma (SSM) or a melanoma with associated nevus and/or said method is used to determine the melanoma stage.

6. The method according to any one of the preceding claims, wherein the measurement of the amount of the microRNA/microRNAs is determined with a method comprising: RNA reverse transcription and / or nucleic acid hybridization and / or nucleic acid amplification and / or a combination thereof.

7. The method according to claim 6, wherein the hybridization of the nucleic acids is carried out using primers and/or probes, each of which is specific and selective for the sequence of one of the microRNAs.

8. The method according to claim 6 or 7, wherein the amplification of the nucleic acids is carried out by quantitative real-time or digital PCR, preferably comprising forward and reverse primers and optionally a probe.

9. The method according to any one of the claims 7 or 8, wherein the probe comprises a sequence complementary to the sequences of the miRNA as defined in claim 1.

## Patentansprüche

1. In-vitro- oder Ex-vivo-Verfahren zum Bestimmen der Prognose und/oder zum Bestimmen des Stadiums eines Melanoms bei einem Individuum, umfassend die folgenden Schritte:
a) Messen der Menge:
i. von mindestens miR-21-5p und miR-146a-5p und wahlweise von miR-l55-5p oder
ii. von mindestens miR-l46a-5p und miR-l55-5p und wahlweise von miR-21-5p oder
iii. von mindestens miR-21-5p und miR-l55-5p und wahlweise von miR-146a-5p
in einer isolierten biologischen Probe, die von dem Individuum erhalten wurde, und
b) Bestimmen des Wertes der Breslow-Dicke mittels Regressionsanalyse,
wobei die Bestimmung des Wertes der Breslow-Dicke in Schritt b) mittels linearer Regressionsanalyse durchgeführt wird, indem die in Schritt a) gemessenen Menge mindestens einer MikroRNA auf eine lineare Regressionsgerade eines Diagramms der MikroRNA-Menge in Bezug auf bekannte Werte der Breslow-Dicke aufgetragen wird, und
wobei der in Schritt b) ermittelte Wert der Breslow-Dicke einen Hinweis auf die Prognose des Patienten gibt und/oder mit dem Grad der Aggressivität des Melanoms korreliert und/oder wobei es sich bei dem Patienten um einen Patienten, bei dem ein Melanom diagnostiziert wurde, oder um einen Patienten, bei dem keine bestimmte Stadieneinteilung vorgenommen wurde, handelt.

2. Verfahren nach Anspruch 1, wobei Schritt a) das Messen der Menge von mindestens miR-2l-5p und miR-l46a-5p und wahlweise von miR-l55-5p umfasst.

3. Verfahren nach den vorhergehenden Ansprüchen, wobei Schritt a) das Messen der Menge der drei MikroRNAs miR-2l-5p, miR-l46a-5p und miR-l55-5p umfasst.

4. Verfahren nach den vorhergehenden Ansprüchen, wobei Schritt a) das Messen der Menge der fünf MikroRNAs miR-2l-5p, miR-146a-5p, miR-l55-5p, miR-363-5p und miR-584-5p umfasst.

5. Verfahren nach einem der vorhergehenden Ansprüche, wobei es sich bei der biologischen Probe um eine Gewebeprobe handelt, wobei es sich bei der Probe vorzugsweise um ein formalinfixiertes und in Paraffin eingebettetes Gewebe oder um frisches Gewebe handelt und/oder wobei es sich bei dem Melanom um ein primäres kutanes Melanom, ein regressionsassoziiertes Melanom, ein superfiziell spreitendes Melanom (SSM) oder ein Melanom mit assoziiertem Nävus handelt und/oder die Methode zur Bestimmung des Melanomstadiums verwendet wird.

6. Verfahren nach einem der vorhergehenden Ansprüche, wobei die Messung der Menge der MikroRNA/MikroRNAs mit einem Verfahren erfolgt, das RNA-Reverse-Transkription und/oder Nukleinsäurehybridisierung und/oder Nukleinsäureamplifikation und/oder eine Kombination davon umfasst.

7. Verfahren nach Anspruch 6, wobei die Hybridisierung der Nukleinsäuren unter Verwendung von Primern und/oder Sonden durchgeführt wird, die jeweils spezifisch und selektiv für die Sequenz von einer der MikroRNAs sind.

8. Verfahren nach Anspruch 6 oder 7, wobei die Amplifikation der Nukleinsäuren durch quantitative Echtzeit-PCR oder digitale PCR durchgeführt wird, vorzugsweise umfassend Vorwärts- und Rückwärtsprimer und wahlweise eine Sonde.

9. Verfahren nach einem der Ansprüche 7 oder 8, wobei die Sonde eine Sequenz umfasst, die zu den Sequenzen der miRNA, wie in Anspruch 1 definiert, komplementär ist.

## Revendications

1. Procédé in vitro ou ex vivo de détermination du pronostic et/ou du stade d'un mélanome chez un sujet comprenant les étapes consistant à :
a) mesurer la quantité de
i. au moins miR-21-5p et miR-146a-5p et éventuellement miR-155-5p ou
ii. au moins miR-146a-5p et miR-155-5p et éventuellement miR-21-5p ou
iii. au moins miR-21-5p et miR-155-p et éventuellement miR-146a-5p
dans un échantillon biologique isolé obtenu du sujet et
b) déterminer la valeur de l'épaisseur de Breslow par analyse de régression,
la détermination de la valeur de l'épaisseur de Breslow dans l'étape b) étant effectuée par analyse de régression linéaire en portant sur un graphe la quantité d'au moins un microARN mesurée dans l'étape a) sur une ligne de régression linéaire d'un graphe de la quantité de microARN par rapport aux valeurs connues de l'épaisseur de Breslow et
la valeur de l'épaisseur de Breslow déterminée dans l'étape b) étant indicatrice du pronostic du patient et/ou corrélée avec le degré d'agressivité du mélanome et/ou le patient étant un patient qui a été diagnostiqué avec un mélanome ou un patient auquel on n'a pas donné un certain stade.

2. Procédé selon la revendication 1, dans lequel l'étape a) comprend la mesure de la quantité d'au moins miR-21-5p et de miR-146a-5p et éventuellement de miR-155-5p.

3. Procédé selon les revendications précédentes, dans lequel l'étape a) comprend la mesure de la quantité des trois microARN : miR-21-5p, miR-146a-5p et miR-155-5p.

4. Procédé selon les revendications précédentes, dans lequel l'étape a) comprend la mesure des cinq microARN : miR-21-5p, miR-146a-5p, miR-155-5p, miR-363-5p et miR-584-5p.

5. Procédé selon l'une quelconque des revendications précédentes, dans lequel l'échantillon biologique est un échantillon de tissu, de préférence ledit échantillon est un tissu fixé à la formaline et inclus dans de la paraffine ou un tissu frais et/ou le mélanome étant un mélanome cutané primaire, un mélanome associé par régression, un mélanome à répartition superficielle (SSM) ou un mélanome avec un naevus associé et/ou ledit procédé est utilisé pour déterminer le stade du mélanome.

6. Procédé selon l'une quelconque des revendications précédentes, dans lequel la mesure de la quantité du/des microARN/microARN est déterminée avec un procédé comprenant la transcription inverse de l'ARN et/ou l'hybridation d'acides nucléiques et/ou l'amplification d'acide nucléique et/ou une de leurs combinaisons.

7. Procédé selon la revendication 6, dans lequel l'hybridation des acides nucléiques est réalisée en utilisant des amorces et/ou des sondes, dont chacune est spécifique et sélective pour la séquence de l'un des microARN.

8. Procédé selon la revendication 6 ou 7, dans lequel l'amplification des acides nucléiques est réalisée par PCR en temps réel ou numérique quantitative, de préférence comprenant des amorces sens et antisens et éventuellement une sonde.

9. Procédé selon l'une quelconque des revendications 7 ou 8, dans lequel la sonde comprend une séquence complémentaire aux séquence du miARN tel que défini dans la revendication 1.
